# EUROPEAN PATENT SPECIFICATION

(11) **EP 1 392 329 B1**
(45) Date of publication and mention of the grant of the patent: **26.07.2006**
(21) Application number: 02733192.5
(22) Date of filing: 19.05.2002
(51) Int. Cl.: A61K 31/715, A61P 1/00, A61P 43/00

(54) **USE OF SULODEXIDE FOR THE TREATMENT OF INFLAMMATORY BOWEL DISEASE**
VERWENDUNG VON SULODEXID ZUR BEHANDLUNG VON REIZDARM
UTILISATION DE SULODEXIDE POUR LE TRAITEMENT D'UNE MALADIE ENTERIQUE INFLAMMATOIRE

(30) Priority: 17.05.2001 US 291667 P
(43) Date of publication of application: 03.03.2004
(73) Proprietor: Keryx Biopharmceuticals Inc., New York NY 10022 (US)
(72) Inventor: SPERO, Michael, 97289 Jerusalem (IL); SHELACH, Noa, Tel-Aviv (IL); LASTER, Morris, 97246 Jerusalem (IL)
(74) Representative: Jump, Timothy John Simon
(86) International application number: PCT/IL2002/000384
(87) International publication number: WO 2003/002129

(56) References cited:
- EP-A- 0 710 483
- WO-A-01/58456
- WO-A-01/58457
- US-A- 3 936 351

## Description

The present invention relates to compositions and kits for preventing and treating inflammatory bowel disease.

### BACKGROUND OF THE INVENTION

### Inflammatory Bowel Disease

Inflammatory Bowel Disease (IBD) is a chronic recurrent inflammatory disease that affects either or both the small intestine and the colon. IBD comprises two major groups: Crohn's disease and ulcerative colitis. The disease affects the small intestine, colon, or both. IBD's cause(s) are not known, but a number of theories have been put forward. Genetic, infectious, immunologic, and even psychological factors have been implicated in a number of studies. For example, 15 to 30 percent of patients have at least one relative that also has IBD. Also, the immune system of patients with IBD have been shown to undergo many changes. Research are being done to determine if a specific gene or group of genes make a person more susceptible to the disease.

Ulcerative colitis (UC) affects the colon and typically gives rise to diarrhea, abdominal cramps, and rectal bleeding, but it may also be accompanied by fatigue, weight loss, appetite loss, loss of body fluids and nutrients, and abdominal pain. A salient feature of UC is that the inflammation of the colon is uniform and continuous. The disease may be limited to the rectum (known as proctitis), may involve part of the colon, or may even involve the entire colon. The surface mucosal cells , including the submucosa and the crypt epithelium, play a role in the inflammatory reaction. As it progresses, epithelial damage ensues along with loss of surface epithelial cells. This give rise to multiple ulcerations. Accordingly, about 85% of UC patients have mild to moderate disease which can be managed without hospitalization. In the remaining 15%, the patients' entire colon are involved and the disease is accompanied by severe bloody diarrhea and systemic symptoms. Toxic dilation of the colon is common among patients with severe UC.

Crohn's Disease (CD) is characterized by inflammation and ulceration occurring deep in the intestinal wall layers. The lower part of the small intestine, the ileum, is one of the most common areas affected. Infrequently, the disease can affect any portion of the gastrointestinal tract. Symptoms include abdominal pain, frequently localized in the lower right side, diarrhea, and weight loss, as well as rectal bleeding and fever. The most common complication of Crohn's disease is intestinal blockage or stricture. This occurs as a result of the disease's thickening of the intestinal walls. Fistulas, which frequently occur around the anus and rectum, are another common complication of the disease. They are abnormal openings that result when ulcers in the intestine create passageways into the surrounding tissues of the bladder, vagina, or the skin.

Early studies have suggested that microthrombi formation in bowel capillaries play a role in IBD pathogenesis. Accordingly, the results support the hypothesis that there exists an endothelial lesion having sustained coagulation activation in IBD patients. Frequently, thromboembolism in various locations is one of IBD's complications. A correlation between thrombotic episodes in IBD patients and decreased levels of some coagulation inhibitors such as antithrombin III, protein C, and protein S was reported in Jorens *et al., Gut,* 61:307-310 (1990). Other studies, in general, have observed a correlation between inflammatory activity and prothrombotic abnormalities. Souto *et al., Digestive Disease and Sciences,* 40:1883-1889 (1995).

Some studies suggest a strong correlation between multiple infarctions of the intestinal mucosa and IBD pathogenesis. Wakefield *et al., Lancet,* 2:1057-1062 (1989). It has also been suggested that decreased blood flow is involved as a prior step in the pathogenesis of inflammation and mucosal ulcerative changes. Leung and Koo, *Dig. Dis. Sci*., 36:727-732 (1991).

A study by Souto *et al.* reported in *Dig. Dis. Sci*., 40:1883-1889 (1995) involved the analysis of the prothrombotic tendency and the role played by the hemostatic system in the etiopathogenesis of IBD. They observed a maintained activation of coagulation in IBD in both active and inactive-phase patients. In particular, the IBD patients exhibited elevated levels of tissue factor pathway inhibitor (TFPI), a natural anti-thrombotic that binds directly and inactivates factor Xa. Also, patients with active disease exhibited higher levels than those with inactive disease, although results of an earlier study does not agree with this finding (Rappaport, *Thromb. Haemostas,* 66:6-15 (1991)).

The correlation between endothelial intestinal injury and IBD has been suggested by a number of observations. For example, clinical, *in vitro,* and animal studies support the theory that elevated concentrations of vW (von Willebrand) factor reflect endothelial cell damage or injury." Blann *et al., Ann Clin. Biochem*., 29:67-71 (1992). Thrombomodulin (TM), a glycoprotein which serves as a receptor for thrombin and which is located on the endothelial cell surface, is a cofactor for thrombin-catalyzed activation of the anticoagulant protease zymogen, protein C. Its plasma levels are elevated in various diseases, which may be attributed to vascular endothelial cell damage. Takano *et al., Blood,* 76:024-2029 (1990).

Other studies also suggest a link between IBD, on the one hand, and microvascular inflammation and hypercoagulable state, on the other. Iglicki and Dupas (AGA Abstracts, *Gastroenterology*, 110:A872) investigated the efficacy of heparin for treating extraintestinal manifestations associated with active IBD. Based on their studies, they suggest it may be possible to treat active UC through the anti-inflammatory and anticoagulant properties of heparin. They also suggest that IBD pathogenesis may arise partly from microvascular inflammation and hypercoagulable state. Brazier *et al*. also suggest that coagulation disorder may play a role in the pathogenesis of UC based on the activation of procoagulant factors and high incidence of thromboembolic events observed in UC. *Gastroenterology*, Abstracts, 110:A872.

Many thromboembolic IBD complications have been reported. These include cerebrovascular disease, internal carotid artery occlusion, portal vein thrombosis, Budd-Chiari, cutaneous gangrene secondary to microvascular thrombosis, retinal vein occlusion, and an increased incidence of ischemic heart disease in UC. These extraintestinal IBD complications mirror a more common underlying hypercoagulable state associated with exacerbations of IBD. IBD has been shown to be accompanied by elevated levels of factors V, VIII, XIII, XIIIA, and fibrinogen, as well as defect in fibrinolysis, decreased levels of antithrombin III, tissue plasminogen activator, protein C, and protein S. A rise in the byproducts of thrombin formation such as fibrinopeptide A, d-dimer, and F1.2 have also been observed. Rectal biopsies in patients with UC and CD have demonstrated the occurrence of mucosal capillary thrombi. In addition, vascular involvement has been observed in both UC and CD.

Certain antiendothelial antibodies, while not being pathogenic themselves, may induce leukocyte attachment to endothelial cells. The intestinal permeability, having been altered, may then become susceptible to microflora. For instance, when blood from UC and CD patients was combined with bacterial endotoxin, intensive formation of fibrin microclot was shown. This microclot formation was not observed in healthy patients or those with acute gastroenteritis without IBD.

It has been posited that radical oxygen metabolites (ROM) could be responsible for most of the inflammatory reaction in IBD. ROM enhance the inflammatory response by activating nuclear factor kB (NF-kB). Also, the levels of proteins, superoxide dismutase, and metallothionein that scavenge ROM are reduced in UC and CD. An important proposed mechanism for the action of 5-aminosalicylates (5-ASA), which is one of the many different types of therapeutic agents used for treating IBD, is associated with its potent ROM-scavenging ability.

The recruitment of leukocytes by cell adhesion molecules, which is induced by interleukin-1 (IL-1), tumor necrosis factor (TNF), and other inflammatory cytokines mediates, mediates the endothelial cell's role in initiating and perpetuating inflammation. In addition, thrombin induces leukocyte adhesion to endothelial cells. The results of many studies are consistent with the finding that IL-1, TNF, and other inflammatory mediators increase prothrombotic factors and contribute substantially to intravascular coagulation. For instance, a pilot study demonstrating the benefits of using anti-TNF antibody in CD suggested that the antibody worked by quickly reversing the hypercoagulable profile induced by TNF.

Surgical procedures for Crohn's disease and ulcerative colitis differ but medical therapy for both are often similar. Medical therapy may include enemas or suppositories for IBD restricted to, for example, the distal colon while oral or intavenous forms must be used if the disease affects the entire colon or the small intestine. Traditionally, IBD therapy has included corticosteroids and aminosalicylates. Recently, various other classes of compounds have been investigated as potential therapeutic agents. These include new steroid preparations, immunosuppressive agents, and cyclosporine.

To assess the efficacy of medical therapy for IBD, a number of parameters are used. These include clinical improvement, clinical remission, endoscopic improvement, histologic improvement, or numerical indices such as the Crohn's Disease Activity Index.

### Conventional and Experimental Therapies for IBD

Steroids have been shown to be efficacious in clinical trials as early as the 1950's for treating active moderate to severe Crohn's disease and ulcerative colitis. Hydrocortisone and prednisolone are commonly used both in the United States and Europe for treating distal ulcerative colitis. Accordingly, they have clinical efficacy rates ranging from about 60% to 80%. Main concerns include systemic side effects and adrenal suppression especially with enema-administered steroids that can have as much as 60% bioavailability. Giller *et al., Am. J. Gastroenterol*. 73: 232-237 (1980). To minimize these concerns, certain steroid preparations such as those having lower bioavailability have been developed.

Aminosalicylates, such as mesalamine (5-aminosalicylic acid) are used in the management of UC. Mesalamines without the sulfa component, such as sulfasalazine are also available. Oral sulfasalazine have been shown to be superior to placebo and similar or superior to steroid enemas in the treatment of mild to moderate UC. Some patients unresponsive to steroid enemas have been shown to achieve remission with 5-aminosalicylate (5-ASA) enemas. Friedman *et al., Am. J. Gastroenterol.* 81: 412-418 (1986); McPhee *et al., Dig. Dis. Sci.* 32: 76S-81S (1987); Guarino *et al., Am. J. Gastroenterol*., 82: 732-737 (1987); Biddle and Miner, *Gastroenterology,* 99: 113-118 (1990). Their value in the treatment of active Crohn's disease is unclear and some patients with UC remain refractory.

Short-chain fatty acids (SCFA), such as butyrate, are the preferred energy substrate for distal colonic epithelial cells and are being tested as experimental therapy. Roediger, *Gut*, 21:793-798 (1980). Compared to patients with Crohn's disease, patients with ulcerative colitis have been shown to have decreased fecal concentrations of SCFA. Vernia *et al., Dig. Dis. Sci*., 33: 1353-1358 (1988). The reported response rates of 56%to 90% are slightly lower than those reported for hydrocortisone or mesalamine enemas. The use of SCFA in IBD therapy is unique in that the presumed mechanism of action is feeding the colon instead of altering its immune response.

Immunosuppresive therapy have become acceptable therapy for IBD states that are difficult to control with traditional therapy such as glucocorticoids and 5-ASA analogs. They are frequently used in treating Crohn's disease, but less so in ulcerative colitis where surgical procedure is the therapy of choice in refractory cases. Indications include treating active disease, steroid dose reduction, remission maintenance, and healing of fistulas. Toxicity associated with azathioprine and 6-MP remains a substantial concern. Some of the side effects include bone marrow suppression, nausea, and pancreatitis.

Cyclosporine, which is widely-used in organ transplant recipients, is another potent immunosuppresant. Results of studies involving the use of cyclosporine in the treatment of fistulas associated with Crohn's disease were shown to be excellent. Improvements as high as 100% have been observed in patients involved in several small studies. Stange *et al., Dig. Dis.* Sci., 34:1387-1392 (1989); Hanauer and Smith, *Am. J. Gastroenterol*., 88:646-649 (1993); Lichtiger, *Mt. Sinai J. Med,* 57:315-319 (1990). But when it comes to the long-term performance of cyclosporine therapy as treatment for severe ulcerative colitis, the results of recent studies have been disappointing.

Methotrexate has been used in a number of uncontrolled trials for Crohn's disease. Results of a recent placebo-controlled trial for steroid-dependent chronically active Crohn's disease showed that methotrexate substantially improved rates of clinical remission, improved scores in the Crohn's disease activity index, and reduced dosages of steroids. Feagan *et al., N. Engl. J. Med,* 332: 292-297 (1995).

Nicotine, which belongs to neither the class of anti-inflammatory or immunosuppresive agents described above, has been studied as a potential IBD therapeutic since it was observed that ulcerative colitis is predominantly a nonsmokel disease and anecdotal reports suggested that smoking cessation exacerbated the symptoms or the resumption of smoking led to an improvement in the patient's condition. Favorable results were obtained from an open trial of nicotine patches (Srivastava *et al., Eur. J. Gastroenterol*., 3:815-818 (1991)) and a randomized crossover trial of nicotine chewing gum (Lashner *et al., Dig. Dis. Sci*., 35:827-832 (1990)) involving patients with active disease. But in maintaining remission of ulcerative colitis, a placebo-controlled trial showed nicotine was ineffective. The role of nicotine therapy in treating ulcerative colitis remains unclear.

Although it has not been confirmed, it has been suspected that infectious agents may be at least partly responsible in the etiology or worsening of IBD. Among antibiotics that have been investigated as possible IBD therapy, metronidazole is an exception. While other antibiotics that have been investigated performed disappointingly, metronidazole in several uncontrolled trials yielded impressive results when used in the treatment of perianal Crohn's disease: practically all patients showed improvements and between 38% to 50% of the patients showed complete healing of fistulas. Bernstein *et al., Gastroenterology*, 79:357-365 (1980); Brandt *et al., Gastroenterology*, 83:383-387 (1982); Jakobovits and Schuster, *Am. J. Gastroenterol*., 79:533-540 (1984). Metronidazole is now considered standard therapy for perianal therapy, but it has not been evaluated in controlled trials. The efficacy of antibiotics in the treatment of ulcerative colitis has not been conclusively demonstrated although studies have shown that ciprofloxacin may provide some benefits in remission maintenance. Turunen et al, *Gastroenterology*, 115:1072-1078 (1998). For now, it is generally considered that the use of antibiotics in ulcerative colitis should be restricted to intravenous antibiotics as adjunctive therapy for severe, refractory colitis. Jarnerot *et al*., *Gastroenterology*, 89:1005-1013 (1985).

Additional therapies include thalidomide and anti-tumor necrosis factor agents such as a monoclonal antibody called cA2 or infliximab (commercially available under the trade name Remicade® ) which is expensive and requires careful monitoring. In addition, there are emerging IBD therapies which include biological agents such as CDP571 (a humanized IgG4 anti-TNF-α antibody), IL-10 (a classic Th2 cytokine which act to suppress production of IL-2 and IFN-γby Th1 cells), IL-11 (a cytokine obtained from mesenchymal cells), ISIS-2302 (an antisense oligonucleotide), anti-α4 antibody (a humanized anti-α4 integrin antibody), and Nissle 1917 (a nonpathogenic *E. coli* strain). These agents are currently undergoing clinical trials. Their safety and efficacy remain to be demonstrated by further studies.

The drawbacks associated with many of the above described therapies are well known. Clearly, improved therapies for IBD are needed.

### Glycosaminoglycans

Heparin is a heterogeneous glycosaminoglycan (GAG) compound and its function may include not only its role as an anticoagulant (through its potentiation of anti-thrombin III and factor Xa) but other functions as well. The range of functions or activities of heparin are directed at reversing the endothelial dysfunction resulting in anti-inflammatory action, endothelial cell interactions, cytokine interaction, radical oxygen metabolites, and intestinal repair. Heparin also reduces ROM generation by stimulated neutrophils and binds superoxide dismutase (SOD) to the endothelial cell. Compared to animal studies, human studies regarding heparin's anti-inflammatory activity are limited. 'Korzenik, *Inflammatory Bowel Diseases,* 3:87-94 (1997).

Murch *et al.* (*The Lancet,* 341:711-714 (1993)) have investigated the distribution and nature of sulphated glycosaminoglycans (GAG's) within normal and inflamed intestines. They found substantial abnormalities of extracellular matrix GAG's which were limited to the mucosa in ulcerative colitis and greatest in the submucosa in CD. Although the study did not address the mechanism leading to the loss of GAG's in IBD, they suggested that the inflammatory disruption of vascular and connective tissue GAG's may be an important pathogenic mechanism, which contributes to the leakage of protein and fluid thrombosis and tissue remodelling seen in IBD.

Sulodexide is a glycosaminoglycan of natural origin extracted from mammalian intestinal mucosa that posses an anticoagulant activity. Sulodexide has a sulfation degree lower than those of heparin. Radhakrishnamurthy B. *et al., Atherosclerosis,* 31:217-229 (1978). In pharmacological models, sulodexide has been shown to be a strong anticoagulant, antithrombotic, and profibrinolytic agent Callas *et al., Semin Thromb. Hemost.,* vol. 19, 49 (1993). A study has also demonstrated that sulodexide therapy is accompanied by less bleeding than when heparin is used. Barbanti *et al., Int. J. Clin. Lab. Res.,* vol. 22,179 (1992); Iacoviello *et al., Thromb. Haemostas.,* vol. 76,1002 (1996). Sulodexide also offers additional advantages such as its ability to improve blood viscosity, lower fibrinogen levels, and increase fibrinolytic activity. Harenberg, *Med. Res. R*ev*.*, 18: 1-20 (1998). In addition, studies indicate a high sulodexide bioavailability after intramuscular and oral administration. *Id*. The preparation of sulodexide is described in U. S. Patent 3,936, 351.

Sulodexide comprises about 80% iduronylglycosaminoglycan sulfate (IGGS), which is a fast-moving heparin fraction, and about 20% dermatan sulfate. The fast moving component, which is determined by its electrophoretic mobility in the barium-propanediamine system, is found in commercial heparin along with a slower moving component. IGGS has a low to medium molecular weight of about 7 kD and a lower anticoagulant activity than the slow moving heparin fraction and unfractionated heparin. Compared to heparin, IGGS has the same dimeric component but with lower amount of iduronic acid-2-O-sulfate and a different amount of glucosamine-acetylated-glucuronic acid dimer.

Sulodexide is marketed in Europe under the trademark VESSEL DUEF® and is prescribed for the treatment of vascular pathologies with thrombotic risk such as peripheral occlusive arterial disease (POAD), healing of venous leg ulcers and intermittent claudication, as described by Harenberg J, *Med. Res. Rev.* vol.18, 1-20 (1998) andCrepaldi G. *et al*., *Atherosclerosis,* 81, 233, (1990), cardiovasculopathies, as described by Tramarin R. *et al. Medical Praxis,* 8,1, (1987), cerebrovasculopathies as described by Sozzi C., *Eur. Rev. Med. Pharmacol Sci*. 6,295, (1984), and venous pathologies of the lower limbs, as described by Cospite M. et al, *Acta Therapeutica,* 18, 149, (1992).

### SUMMARY OF THE INVENTION

The present invention is directed to a pharmaceutical composition comprising from 60% to 90% iduronylglycosaminoglycan sulfate and between 10% to 40% dermatan sulfate, or a pharmaceutically acceptable salt, solvate, hydrate, or clathrate thereof, and an active ingredient selected from:
a) steroids, aminosalicylates, short-chain fatty acids, thioguanine derivatives, antibiotics, biological agents, antidepressants, pain relievers, and mixtures thereof, or
b) mesalamine, hydrocortisone, prednisolone, azathioprine, 6-MP, cyclosporine, methotrexate, metronidazole, ciprofloxacin, nicotine, penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin, imipramine, amitriptyline, desipramine, nortriptyline, doxepin, protriptyline, trimipramine, maprotiline, amoxapine, uazodone, bupropion, chlomipramine, fluoxetine, sertraline, paroxetine, fluvoxamine, nefazadone, venlafaxine, mirtazapine, monoamine oxidase inhibitors, phenelzine, tranylcypromine, acetaminophen, ibuprofen, and acetylsalicylic acid, naproxen, fenoprofen, indomethacin, ketorolac, thalidomide, infliximab, CDP571, IL-10, IL-11, ISIS-2302, anti-α4-antibody, Nissle 1917 and mixtures thereof,
for use in a method for preventing, reducing, or eliminating one or more of symptoms inflammatory bowel disease wherein said pharmaceutical composition is to be administered to a patient in need of inflammatory bowel disease treatment in an amount sufficient to inhibit, reduce, or eliminate one of more symptoms of inflammatory bowel disease. In a particular embodiment, the composition comprises sulodexide or a pharmaceutically acceptable salt, solvate, hydrate, or clathrate thereof. Examples of IBD include Crohn's disease and ulcerative colitis.

In another embodiment, the present invention is directed to a pharmaceutical composition comprising from 60% to 90% iduronylglycosaminoglycan sulfate and between 10% to 40% dermatan sulfate, or a pharmaceutically acceptable salt, solvate, hydrate, or clathrate thereof, and one or more additional active ingredients selected from:
a) steroids, aminosalicylates, short-chain fatty acids, thioguanine derivatives, antibiotics, biological agents, antidepressants, pain relievers, and mixtures thereof, or
b) mesalamine, hydrocortisone, prednisolone, azathioprine, 6-MP, cyclosporine, methotrexate, metronidazole, ciprofloxacin, nicotine, penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin, imipramine, amitriptyline, desipramine, nortriptyline, doxepin, protriptyline, trimipramine, maprotiline, amoxapine, trazodone, bupropion, chlomipramine, fluoxetine, serttaline, paroxetine, fluvoxamine, nefazadone, venlafaxine, mirtazapine, monoamine oxidase inhibitors, phenelzine, tranylcypromine, acetaminophen, ibuprofen, and acetylsalicylic acid, naproxen, fenoprofen, indomethacin, ketorolac, thalidomide, infliximab, CDP571, IL-10, IL-11, ISIS-2302, anti-α4-antibody, Nissle 1917 and mixtures thereof,
for use in a method of preventing, reducing, or eliminating one or more symptoms of inflammatory bowel disease and wherein all active ingredients are to be administered to a patient in an amount sufficient to inhibit, reduce, or eliminate one or more symptoms of inflammatory bowel disease.

In a further embodiment, the present invention is directed to a use of a composition comprising from 60% to 90% iduronylglycosaminoglycan sulfate or a pharmaceutically acceptable salt, solvate, hydrate or clathrate thereof, and between 10% to 40% dermatan sulfate or a pharmacuatically acceptable salt, solvate, hydrate or dathrate thereof, for the preparation of a medicament for inhibiting, reducing or eliminating one or more symptoms of inflammatory bowel disease.

In yet another embodiment, the present invention is directed to a kit comprising a first pharmaceutical composition which comprises from 60% to 90% iduronylglycosaminoglycan sulfate or a pharmaceutically acceptable salt, solvate, hydrate, or clathrate thereof, and between 10% to 40% dermatan sulfate, or a pharmaceutically acceptable salt, solvate, hydrate, or clathrate thereof, and
a second pharmaceutical composition which comprises one or more additional active ingredients,
for use in a method of preventing, reducing or eliminating one or more symptoms of inflammatory bowel disease in a patient, wherein said first and second pharmaceutical compositions are to be administered to said patient in an amount sufficient to inhibit, reduce or eliminate one or more symptoms of inflammatory bowel disease.

In an aspect of this embodiment, the administration of the first and second pharmaceutical composition is temporally spaced apart by at least about two minutes.

The therapeutically or prophylactically effective amount of an active ingredient ranges from about 20 L.R.U. (lipoprotein lipase releasing unit) to about 100,000 L.R.U. per day, depending on the type of administration. For oral administration, the therapeutically effective amount of the active ingredient may be several times greater than that for parenteral administration. For example, the amount of the active ingredient may range from about five to ten times greater than that for intravenous or subcutaneous administration.

The mode of administration may be oral, mucosal (preferably rectal), parenteral, or transdermal.

The present invention can be more fully explained by reference to the following detailed description and illustrative examples.

### DETAILED DESCRIPTION OF THE INVENTION

Sulodexide has been shown to have strong anticoagulant, antithrombotic, and profibrinolytic activities. Compared to heparin, at least one study has shown that less bleeding accompanies solodexide therapy. Studies also show that sulodexide offers high bioavailability after intramuscular and oral administration. Sulodexide is available commercially under the trademark VESSEL DUE F® , and it can be made by the methods disclosed in U.S. Patent 3,936,351.

Without being limited by theory, it is believed that sulodexide may achieve its therapeutic or prophylactic action in IBD by being involved, directly or indirectly, with any of the following: (a) interference with leukocyte attachment to endothelial cells and their migration to colonic mucosa, both by inhibiting thrombin deposition thereby activating neutrophils, and by blocking the effects of L- and P-selectin, which are particularly elevated in IBD; (b) inhibition not only of phospholipase A₂ but also of neutrophil elastase thereby blocking neutrophil diapedesis across the endothelium; (c) interference in the inflammatory process by blocking several key inflammatory mediators; (d) antagonizing platelet activating factor, which is elevated in IBD and a mediator of prostaglandin and leukotriene generation; (e) protecting against anti-Ig-E-mediated histamine release and inhibits mast cell degranulation; (f) minimizing harmful effects of inflammatory cytokines and promoting endothelial cell repair and healing; (g) limiting the adverse effects of TNF, a cytokine that plays a major role in CD by promoting release of TNF-binding protein 1 and binding TNF directly; (h) binding of a number of growth factors, particularly the fibroblast growth factor

(FGF) family, and protecting them from degradation and potentiating their activity; (i) in combination with acidic FGF, substantial restriction of the endothelial cell response to IL-1 (j); release of diamine oxidase from enterocytes thereby regulating the levels of growth associated polyamines; and (k) interference with helper-T cells' production of proteins such as cytokines which give rise to intestinal inflammation and damage.

A first embodiment of the invention therefore comprises a pharmaceutical composition for use in a method of preventing IBD which comprises from 60% to 90% iduronylglycosaminoglycan sulfate and from 10% to 40% dermatan sulfate, or a pharmaceutically acceptable salt, solvate, hydrate or clathrate thereof and an active ingredient selected from:
a) steroids, aminosalicylates, short-chain fatty acids, thioguanine derivatives, antibiotics, biological agents, antidepressants, pain relievers, and mixtures thereof, or
b) mesalamine, hydrocortisone, prednisolone, azathioprine, 6-MP, cyclosporine, methotrexate, metronidazole, ciprofloxacin, nicotine, penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin, imipramine, amitriptyline, desipramine, nortriptyline, doxepin, protriptyline, trimipramine, maprotiline, amoxapine, trazodone, bupropion, chlomipramine, fluoxetine, sertraline, paroxetine, fluvoxamine, nefazadone, venlafaxine, mirtazapine, monoamine oxidase inhibitors, phenelzine, tranylcypromine, acetaminophen, ibuprofen, and acetylsalicylic acid, naproxen, fenoprofen, indomethacin, ketorolac, thalidomide, infliximab, CDP571, IL-10, IL-11, ISIS-2302, anti-α4-antibody, Nissle 1917 and mixtures thereof,
wherein said pharmacautical composition is to be administered in an amount sufficient to alleviate, prevent, or eliminate one or more underlying causes or symptoms in an IBD patient.

In a preferred embodiment of the invention, said pharmaceutical composition comprises sulodexide, or a pharmaceutically acceptable salt, solvate, hydrate, or clathrate thereof, which is to be administered in an amount sufficient to alleviate or prevent one or more symptoms or complications of IBD. A typical pharmaceutical composition further comprises a pharmaceutically acceptable excipient or diluent.

The pharmaeutical compositions of the invention thus contain one or more additional active ingredient, wherein the additional active ingredient is selected from steroids, aminosalicylates, short-chain fatty acids, biological agents, and antibiotics, as well as antidepressants and pain-relievers. As used herein, the term"steroids"encompass corticosteroids, glucocorticoids, and other steroids or steroid-like compounds that may be used to alleviate or treat symptoms or complications of IBD such as inflammation.

The medicament according to the present invention can also further comprise an active ingredient selected from:
a) steroids, aminosalicylates, short-chain fatty acids, thioguanine, derivatives, antibiotics, biological agents, antidepressants and pain relievers, and mixtures thereof, or
b) mesalamine, hydrocortisone, prednisolone, azathioprine, 6-MP, cyclosporine, methotrexate, metronidazole, ciprofloxacin, nicotine, penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin, imipramine, amitriptyline, desipramine, nortriptyline, doxepin, protriptyline, trimipramine, maprotiline, amoxapine, trazodone, bupropion, chlomipramine, fluoxetine, sertraline, paroxetine, fluvoxamine, nefazadone, venlafaxine, mirtazapine, monoamine oxidase inhibitors, phenelzine, tranylcypromine, acetaminophen, ibuprofen, and acetylsalicylic acid, naproxen, fenoprofen, indomethacin, ketorolac, thalidomide, infliximab, CDP571, IL-10, IL-11, ISIS-2302, anti-α4-antibody, Nissle 1917 and mixtures thereof.

Another embodiment of an invention is a pharmaceutical composition comprising from 60% to 90% iduronylglycosaminoglycansulfate, between 10% to 40% dermatan sulfate, and one or more additional active ingredient and an active ingredient selected from:
a) steroids, aminosalicylates, short-chain fatty acids, thioguanine derivatives, antibiotics, biological agents, antidepressants, pain relievers, and mixtures thereof, or
b) mesalamine, hydrocortisone, prednisolone, azathioprine, 6-MP, cyclosporine, methotrexate, metronidazole, ciprofloxacin, nicotine, penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin, imipramine, amitriptyline, desipramine, nortriptyline, doxepin, protriptyline, trimipramine, maprotiline, amoxapine, trazodone, bupropion, chlomipramine, fluoxetine, sertraline, paroxetine, fluvoxamine, nefazadone, venlafaxine, mirtazapine, monoamine oxidase inhibitors, phenelzine, tranylcypromine, acetaminophen, ibuprofen, and acetylsalicylic acid, naproxen, fenoprofen, indomethacin, ketorolac, thalidomide, infliximab, CDP571, IL-10, IL-11, ISIS-2302, anti-α4-antibody, Nissle 1917 and mixtures thereof for use in a method of inhibiting, reducing, or eliminating symptoms or complications of inflammatory bowel disease wherein said pharmaceutical composition is to be administered and wherein the amount of all active ingredients is sufficient to inhibit, reduce, or eliminate one or more, symptoms, of inflammatory bowel disease.

In another preferred embodiment the invention is a kit comprising:
a first pharmaceutical composition which comprises from 60% to 90% iduronylglycosaminoglycan sulfate or a pharmaceutically acceptable salt, solvate, hydrate, or clathrate thereof, and between 10% to 40% dermatan sulfate, or a pharmaceutically acceptable salt, solvate, hydrate, or clathrate thereof, and a second pharmaceutical composition which comprises one or more additional active ingredients,
for use in a method of preventing, reducing or eliminating one or more symptoms of inflammatory bowel disease in a patient, wherein said first and second pharmaceutical compositions are to be administered to said patient in an amount sufficient to inhibit, reduce or eliminate one or more symptoms of inflammatory bowel disease symptoms or complications of inflammatory bowel disease.

The administration of the first and second pharmaceutical composition is preferably temporally spaced apart by at least about two minutes.

Particular examples of active ingredients that may be administered in conjunction with sulodexide or related glycosaminoglycans are mesalamine, azathioprine, 6-MP, cyclosporine, methotrexate, metronidazole, ciprofloxacin, thalidomide, and nicotine. Examples of antibiotics that may be administered as additional active ingredient for treating IBD include penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin. Examples of antidepressants that may be used according to the method of the invention as additional active ingredient include imipramine, amitriptyline, desipramine, nortriptyline, doxepin, protriptyline, trimipramine, maprotiline, amoxspine, trazodone, bupropion, chlomipramine, fluoxetine, sertraline, paroxetine, fluvoxamine, nefazadone, venlafaxine, mirtazapine, monoamine oxidase inhibitors, phenelzine, and tranylcypiomine. Examples of pain-relievers that may be used as additional active ingredient include acetaminophen, ibuprofen, acetylsalicylic acid, naproxen, fenoprofen, indomethacin, and ketorolac. Examples of biological agents for use in the methods of the invention include infliximab, CDP571, IL-10, IL-11, ISIS-2302, anti-α4 antibody, and Nissle 1917.

Administration may be oral, mucosal, parenteral, or transdermal. The pharmaceutical composition may also be administered through the use of an enema or suppository. Generally, the dosage will vary depending on the severity of the disease, mode of administration, and the patient's general condition, age, and weight.

Preferably, the pharmaceutical composition is in the form of an oral preparation. Because of their ease of administration, tablets and capsules are preferred and represent the most advantageous oral dosage unit form, in which case solid pharmaceutical excipients are employed. If desired, tablets may be coated by standard aqueous or nonaqueous techniques.

Preferably, the oral pharmaceutical compositions of the present invention may be administered in single or divided doses, from one to four times a day. The oral dosage forms may be conveniently presented in unit dosage forms and prepared by any methods well known in the art of pharmacy.

Preferably, the therapeutically or prophylactically effective amount of an active ingredient ranges from about 20L. R.U. (lipoprotein lipase releasing unit) to about 100,000 L. R. U. daily, depending on the type of administration. For oral administration, the therapeutically effective amount of the active ingredient may be several times greater than that for parenteral administration. The amount of the orally administered active ingredient may range from about five to ten times greater than that for intravenous or subcutaneous administration. Preferably, the amount of pharmaceutical composition used daily in the present invention ranges from about 20 L. R. U. to about 1,000 L. R. U. for parenteral administration, about 200 L. R. U. to about 10,000 L. R. U. for oral administration, and about 2, 000 L. R. U. to about 100, 000 L. R. U. for rectal administration. The pharmaceutical composition preferably comprises VESSEL DUE F®. Preferred solid dosage forms of the pharmaceutical compositions are tablets or capsules, which are either coated or uncoated. The oral pharmaceutical compositions may be administered in single or divided dosage from one to four times a day, and preferably range from 50 milligrams ("mg") (500 L. R. U.) a day to about 1000 mg (10,000 L. R. U.) a day such as, for example, 50 mg/day, 100 mg/day, 150 mg/day, 200 mg/day, 300 mg/day, 400 mg/day, 500 mg/day. Preferably, when the pharmaceutical compositions are administered rectally, the dosage should be about 10 times higher than that used for oral administration, i.e., from about 500 mg to about 10 g. Preferably, the medicament of the present invention is to be administered in order to provide 20 lipoprotein lipase reducing agent units (L.R.U.) to 100,000 L.R.U. of said composition, per day. Alternatively, the medicament is to be administered in order to provide 20 L.R.U. to 100,000 L.R.U. of sulodexide, or a pharmaceutically acceptable salt, solvate, hydrate or clathrate thereof, per day.

Even more preferably, the medicament is to be administered: parenterally, in order to provide 20 L.R.U. to 1,000 L.R.U., of said composition, per day; orally, in order to provide 200 L.RU. to 10,000 L.R.U., of said composition, per day; rectally, in order to provide 2000 L.R.U. to 100,000 L.R.U., of said composition, per day; parenterally, in order to provide 2 mg to 100 mg of said composition, per day; orally, in order to provide 20 mg to 1000 mg of said composition, per day; or rectally, in order to provide 200 mg to 10,000 mg of said composition, per day.

The compositions and medicaments of the invention include sulodexide, or its pharmaceutically acceptable salt, solvate, hydrate, or clathrate, which can be administered in combination with one or more additional active ingredients. The invention includes the simultaneous or sequential administration of two or more active ingredients. If the administration is sequential, the separate administrations may be temporally spaced apart by about at least two minutes or more. Temporal spacing of the action or effects of the active ingredients may also be achieved by using, for each active ingredient, at least one different carrier, excipient, or solid dosage coating, so as to cause a differential rate of release of each of the active ingredient into the body. Alternatively, at least one of the active ingredients may be administered in a controlled-release preparation, or each of the ingredients may be administered in a different controlled-release preparation each of which has its own release rate. Examples of second active ingredients include, but are not limited to, steroids, aminosalicylates, short-chain fatty acids, antibiotics, biological agents, antidepressants, and pain-relievers. Particular examples of active ingredients that may be used in conjunction with sulodexide are mesalamine, azathioprine, 6-MP, cyclosporine, methotrexate, metronidazole, ciprofloxacin, thalidomide, and nicotine.

Preferred pharmaceutical compositions and unit dosage forms for use in the invention comprise a pharmaceutically acceptable excipient or diluent. Pharmaceutical unit dosage forms of this invention are suitable for oral, mucosal (*e*.*g*., nasal; sublingual, vaginal, buccal, or rectal), parenteral (*e*.*g*., subcutaneous, intravenous, bolus injection, intramuscular, or intraarterial), or transdermal administration to a patient.

Proper diagnosis of IBD should be made prior to the treatments of the invention to rule out other possible diseases that may exhibit similar symptoms, such as cancer, infections, or irritable bowel syndrome. The diagnosis preferably includes a thorough physical examination of the patient, which includes blood tests and stool analysis. Particular attention should be paid to the presence of common IBD symptoms such as bloody diarrhea, fatigue, and weight loss. The procedures preferably used in the diagnosis include well-known procedures such as sigmoidoscopy, colonoscopy, upper and lower gastrointestinal barium x-ray, and computed tomography scans.

### Pharmaceutical Compositions and Dosage Forms Useful in the Invention

Pharmaceutical compositions and dosage forms which may be used in the invention comprise one or more of the active ingredients disclosed herein. Pharmaceutical compositions and dosage forms of the invention typically also comprise one or more pharmaceutically acceptable excipients or diluents.

Single unit dosage forms of the invention are suitable for oral, mucosal (*e*.*g*., nasal, sublingual, vaginal, buccal, or rectal), parenteral (*e*.*g*., subcutaneous, intravenous, bolus injection, intramuscular, or intraarterial), or transdermal administration to a patient. Examples of dosage forms include tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g*., nasal sprays or inhalers); gels; liquid dosage forms suitable for oral or mucosal administration to a patient, including suspensions (*e*.*g*., aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g*., crystalline or amorphous solids) that can be reconstituted to provide liquid dosage forms suitable for parenteral administration to a patient.

The composition, shape, and type of dosage forms of the invention will typically vary depending on their use. For example, a dosage form used in the acute treatment of inflammation or a related disorder may contain larger amounts of one or more of the active ingredients it comprises than a dosage form used in the chronic treatment of the same disease. Similarly, a parenteral dosage form may contain smaller amounts of one or more of the active ingredients it comprises than an oral dosage form used to treat the same disease or disorder. These and other ways in which specific dosage forms encompassed by this invention will vary from one another will be readily apparent to those skilled in the art. *See, e.g., Remington's Pharmaceutical Sciences,* 18th ed., Mack Publishing, Easton PA (1990).

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients can be accelerated by the use of excipients such as lactose, or exposure to water. Consequently, this invention encompasses pharmaceutical compositions and dosage forms that contain little, if any, lactose other mono- or di-saccharides. As used herein, the term "lactose-free" means that the amount of lactose present, if any, is insuffcient to substantially increase the degradation rate of an active ingredient.

Lactose-free compositions of the invention can comprise excipients that are well known in the art and are listed, for example, in the U.S. Pharmacopeia (USP) SP (XXI)/NF (XVI). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

One may use oil-water emulsions, liposomes, and calcium-binding molecules to increase the absorption of sulodexide. In these approaches, one may incorporate agents in the pharmaceutical compositions agents such as acids, oils, EDTA, surfactants, amines, amides, amino acids, or thickening agents. The thickeners used comprise polymers such as vinyl polymers, gums such as gum arabic and xanthan gum, pectins, starch, casein, alginates, carrageenans, hydroxyethylcellulose, methylcellulose, or polyvinylpyrrolidone. The surfactants include anionic surfactants containing carboxylate, sulfonate and sulfate ions such as sodium lauryl sulfate, cationic agents such as amine salts and quaternary ammonium salts, amphoteric agents such as those containing carobxylate or phosphate groups as the anion and amino or quaternary ammonium groups as the cations, peptides, proteins, betaines, polyoxyethylenic alcohols, phospholipids such as lecithin and cephalins, and nonoionic agents such as long-chain fatty acids and their derivatives, glyceryl esters, fatty acid esters of fatty alcohols such as propylene glycol, sorbitan, sucrose, and cholesterol. Other types of surfactants known in the art may also be used.

Because water can facilitate the degradation of some compounds, this invention further encompasses anhydrous pharmaceutical compositions and dosage forms comprising active ingredients. The effect of water on a formulation can be significant since moisture and/or humidity are commonly encountered during manufacture, handling, packaging, storage, shipment, and use of formulations. As a means of simulating long-term storage in order to determine characteristics such as shelf-life or the stability of formulations over time, the addition of water (*e*.*g*., 5%) is widely accepted in the pharmaceutical arts. *See, e.g.,* Jens T. Carstensen, *Drug Stability: Principles & Practice*, 2d. Ed.; Marcel Dekker, NY, NY, 1995, pp. 379-80.

The pharmaceutical compositions may be prepared by any methods well known in the art of pharmacy, but all methods include the step of bringing into association one or more active ingredient(s) with the carrier. In general, the compositions are prepared by uniformly and intimately admixing the active ingredients with liquid carriers or finely divided solid carriers or both, and then, if necessary, shaping the product into the desired presentation. Oral solid preparations are preferred over oral liquid preparations. One preferred oral solid preparation is tablets, but the most preferred oral solid preparation is capsules.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water so that they can be included in suitable formulary kits. Examples of suitable packaging include hermetically sealed foils, plastics, unit dose containers (*e.g*., vials), blister packs, and strip packs.

The invention further encompasses pharmaceutical compositions and dosage forms that comprise one or more compounds that reduce the rate by which an active ingredient will decompose. Such compounds, which are referred to herein as "stabilizers," include; but are not limited to, antioxidants such as ascorbic acid, pH buffers, or salt buffers.

Like the amounts and types of excipients, the amounts and specific types of active ingredients in a dosage form may differ depending on factors such as the route by which it is to be administered to patients. Other factors include the patient's general condition, age, and weight, as well as the severity of the disease: Preferred dosage forms of the invention comprise sulodexide, or a pharmaceutically acceptable salt, solvate, clathrate, or hydrate thereof in an amount ranging from about 20 L.R.U. to about 100,000 L.R.U.

### Oral Dosage Forms

Pharmaceutical compositions of the present invention suitable for oral administration may be presented as discrete pharmaceutical unit dosage forms, such as capsules, cachets, soft elastic gelatin capsules, tablets, caplets, or aerosols sprays, each containing a predetermined amount of the active ingredients, as a powder or granules, or as a solution or a suspension in an aqueous liquid, a non-aqueous liquid, an oil-in-water emulsion, or a water-in-oil liquid emulsion. Dosage forms such as oil-in-water emulsions typically comprise surfactants such as anionic phosphate ester or lauryl sulfates, but other types of surfactants such as cationic or nonionic surfactants may be used in the compositions of the present invention. *See generally, Remington's Pharmaceutical Sciences,* 18th ed., Mack Publishing, Easton PA (1990).

Pharmaceutical compositions of the present invention suitable for oral administration may be formulated as a pharmaceutical composition in a soft elastic gelatin capsule unit dosage form by using conventional methods well known in the art. *See, e.g.,* Ebert, *Pharm. Tech,* 1(5):44-50 (1977). Pharmaceutical compositions in the form of capsules or tablets coated by an enterosoluble gastroresistant film and which contains a lyophilisate consisting of glycosaminoglycan, a thickening agent, and a surfactant have been previously described in U.S. Patent No. 5,252,339. Soft elastic gelatin capsules have a soft, globular gelatin shell somewhat thicker than that of hard gelatin capsules, wherein a gelatin is plasticized by the addition of plasticizing agent, *e.g*., glycerin, sorbitol, or a similar polyol. The hardness of the capsule shell may be changed by varying the type of gelatin used and the amounts of plasticizer and water. The soft gelatin shells may contain a preservative, such as methyl- and propylparabens and sorbic acid, to prevent the growth of fungi. The active ingredient may be dissolved or suspended in a liquid vehicle or carrier, such as vegetable or mineral oils, glycols, such as polyethylene glycol and propylene glycol, triglycerides, surfactants, such as polysorbates, or a combination thereof.

Typical oral dosage forms of the invention are prepared by combining the active ingredient(s) in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, water, glycols, oils, alcohols, flavoring agents, preservatives, and coloring agents. Examples of excipients suitable for use in solid oral dosage forms (*e*.*g*., powders, tablets, capsules, and caplets) include starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include binders, fillers, disintegrants, and lubricants. Binders suitable for use in pharmaceutical compositions and dosage forms include corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e*.*g*., Nos. 2208, 2906, 2910), microcrystalline cellulose, and mixtures thereof.

Suitable forms of microcrystalline cellulose include, the materials sold as AVICEL® PH-101, AVICEL® PH-103 AVICEL® RC-581, AVICEL® PH-105. (available from FMC Corporation, American Viscose Division, Avicel Sales, Marcus Hook, PA), and mixtures thereof. An specific binder is a mixture of microcrystalline cellulose and sodium carboxymethyl cellulose sold as AVICEL® RC-581. Suitable anhydrous or low moisture excipients or additives include AVICEL® PH-103 and Starch 1500 LM.

Examples of fillers suitable for use in the pharmaceutical compositions and dosage forms disclosed herein include talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Pharmaceutical stabilizers may also be used to stabilize the compositions of the invention. Acceptable stabilizers include to L-cysteine hydrochloride, glycine hydrochloride, malic acid, sodium metabisulfite, citric acid, tartaric acid and L-cysteine dihydrochloride.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant

Disintegrants that can be used in pharmaceutical compositions and dosage forms of the invention include agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms of the invention include calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e*.*g*., peanut oil, cottonseed oil, sunflower oil, sesame oil, olive oil, corn oil, and soybean oil), zinc stearate, ethyl oleate, ethyl laureate, agar, and mixtures thereof Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### Delayed Release Dosage Forms

Active ingredients of the invention can be administered by controlled release means or by delivery devices that are well known to those of ordinary skill in the art. Examples include those described in U.S. Patent Nos.: 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposoines, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects. Further, when it is desired to temporally space the release, and hence the effect, of two or more active ingredients used in the method of the invention, it is possible to use: (a) a controlled-release preparation for the release of at least one of the active ingredients; or (b) two or more controlled-release preparations having different release coefficients, for the separate release of each active ingredient.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### -Parenteral Dosage Forms

Parenteral dosage forms can be administered to patients by various routes including subcutaneous, intravenous (including bolus injection), intramuscular, and intraarterial. Because their administration typically bypasses patients' natural defenses against contaminantss, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to those skilled in the art. Examples include Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms of the invention.

### Transdermal, Topical, Mucosal, and Rectal Dosage Forms

Transdermal, topical, and mucosal dosage forms of the invention include ophthalmic solutions, sprays, aerosols, creams, lotions, ointments, gels, solutions, emulsions, suspensions, enemas, suppositories, or other forms known to one of skill in the art. *See, e.g., Remington's Pharmaceutical Sciences*, 16th and 18th eds., Mack Publishing, Easton PA (1980 & 1990); and *Introduction to Pharmaceutical Dosage Forms*, 4th ed, Lea & Febiger, Philadelphia (1985). Further, transdermal dosage forms include "reservoir type" or "matrix type" patches, which can be applied to the skin and worn for a specific period of time to permit the penetration of a desired amount of active ingredients.

Suitable excipients (*e*.*g*., carriers and diluents) and other materials that can be used to provide transdermal, topical, and mucosal dosage forms encompassed by this invention are well known to those skilled in the pharmaceutical arts, and depend on the particular tissue to which a given pharmaceutical composition or dosage form will be applied. With that fact in mind, typical excipients include water, acetone, ethanol, ethylene glycol, propylene glycol, butane-1,3-diol, isopropyl myristate, isopropyl palmitate, mineral oil, and mixtures thereof to form lotions, tinctures, creams, emulsions, gels or ointments, which are non-toxic and pharmaceutically acceptable. Moisturizers or humectants can also be added to pharmaceutical compositions and dosage forms if desired. Examples of such additional ingredients are well known in the art. *See, e.g., Remington's Pharmaceutical Sciences,* 16th and 18th eds., Mack Publishing, Easton PA (1980 & 1990).

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients, particularly for the tissues of the colon. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent Different salts, hydrates or solvates of the active ingredients can be used to further adjust the properties of the resulting composition.

### Kits

The active ingredients of the invention may or may not be administered to a patient at the same time or by the same route of administration. Therefore, the methods of the invention encompass kits which, when used by the medical practitioner, can simplify the administration of appropriate amounts of active ingredients to a patient.

A typical kit of the invention comprises a unit dosage form of sulodexide, or a pharmaceutically acceptable salt, solvate, hydrate, or clathrate thereof, and a unit dosage form of at least one additional active ingredient. Examples of additional active ingredients that may be used in conjunction with sulodexide or related glycosaminoglycans, include steroids, aminosalicylates, short-chain fatty acids, antibiotics, and biological agents, as well as antidepressants and pain-relievers. Other active ingredients that may be used in conjunction with sulodexide are mesalamine, azathioprine, 6-MP, cyclosporine, methotrexate, metronidazole, ciprofloxacin, and nicotine. Examples of antibiotics that may be used as additional active ingredient for treating IBD include penicillins, cephalosporins, vancomycin, bacitracin, macrolides, lincosamides, chloramphemcol, tetracyclines, aminoglycosides, mupirocin, sulfonamides, trimethoprim, rifampin, metronidazole, quinolones, novobiocin, polymyxins, gramicidin. Examples of antidepressants that may be used as additional active ingredient include imipramine, amitriptyline, desipramine, nortriptyline, doxepin, protriptyline, trimipramine, mapmtiline, amoxapine, trazodone, bupropion, chlomipramine, fluoxetine, sertraline, paroxetine, fluvoxamine, nefazadone, venlafaxine, mirtazapine, monoamine oxidase inhibitors, phenelzine, and tranylcypromine. Examples of pain-relievers that may be used as additional active ingredient include acetaminophen, ibuprofen, acetylsalicylic acid, naproxen, fenoprofen, indomethacin, and ketorolac. Examples of biological agents for use in the methods of the invention include, but are not limited to, infliximab, CDP571, IL-10, IL-11, ISIS-2302, anti-α4 antibody, and Nissle 1917.

Kits of the invention can further comprise devices that are used to administer the active ingredients. Examples of such devices include syringes, drip bags, patches, inhalers, enemas, and dispensers for the administration of suppository formulations.

Kits of the invention can further comprise pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

The following series of examples are presented by way of illustration.

### EXAMPLES

### Example 1

### MATERIALS AND METHODS

Animals: Normal inbred, male Sabra rats (200-300 gr.) maintained on standard laboratory chow and kept in 12 hr light/dark cycles.

Induction of colitis: TNBS-colitis is induced by rectal instillation of 2,4,6-trinitrobenzene sulfonic acid (TNBS) at 25mg/rat, dissolved in 1 ml of 50% ethanol.

Clinical assessment of colitis: Diarrhea is monitored daily throughout the study.

Macroscopic score of colitis: Ten days after colitis induction, the rats are sacrificed and their colon is removed to assess the extent of colitis. The percentage of the total injured colonic wall and colon weight is measured. Four additional parameters are determined: (1) degree of colonic ulcerations; (2) intestine and peritoneal adhesions; (3) wall thickness; and (4) degree of mucosal edema. Each parameter is graded on a scale from 0 (completely normal) to 4 (most severe) by two experienced blinded examiners.

Grading of histological lesions: For histological evaluation of inflammation, distal colonic tissue (last 10 cm) is removed and fixed in 10% formaldehyde. Five paraffin sections from each rat are then stained with hematoxylin-eosin using standard techniques. The degree of inflammation on microscopic cross sections of the colon is graded semi-quantitatively from 0 to 4 as follows:
Grade 0: normal with no signs of inflammation;
Grade 1: very low level of leukocyte infiltration;
Grade 2: low level of leukocyte infiltration;
Grade 3: high level of infiltration with high vascular density, and thickening of the bowel wall; and
Grade 4; transmural infiltrates with loss of goblet cells, high vascular density, wall thickening, and disruption of normal bowel architecture.

Grading is performed by two experienced blinded pathologists.

Serum TGFβ1 and IFNγ levels: TGFβ1 and IFNγ levels are measured by a "sandwich" ELISA using Genzyme Diagnostics kits (Genzyme Diagnostics, MA, USA) according to the manufacturer's instructions. Serum levels are measured in all rats from tolerized and non-tolezized control rats 10 days after colitis induction.

The first group of rats, which comprises the control group, are not treated with sulodexide solution. Each member of the second group is orally administered with sulodexide solution twice a day, once in the morning and once in the evening, for eleven days.

The results for both the control group and the treated group, are presented according to the severity of the colic alterations in terms of preassigned numerical value.

### Example 2

Patients with Crohn's disease are investigated. All patients are well-fed and well-hydrated at the time of the study. Diagnoses are conducted through endoscopic, radiologic, and histologic studies. Patients are characterized as having inactive, mild, moderately or severely active disease. Patients having inactive disease are defined as less than two bowel movements each day with no blood or mucus per rectum, and no visible inflammation as determined through sigmoidoscopy. Patients having mild disease are defined as less than four bowel movements per day accompanied by blood and mucus, and outer inflammation as shown by sigmoidoscopy. Patients having moderately or severely active disease are defined as greater than four bowel movements per day associated with cramping, with evidence of fever or systemic signs of the disease found only in severe cases. Body mass index (BMI) is determined as weight (kg)/height (m)².

Tissue samples, which are freshly resected from the patients are obtained for evidence of inflammation on histology.

The patients are divided into equal groups and are administered orally with about 50 mg, 100 mg or 200 mg per day of sulodexide such as VESSEL DUE F® for about 10 to 16 weeks. Alternatively, patients are divided into equal groups and rectally receive 500 mg, 1000 mg or 2000 mg of sulodexide daily for about 10 to 16 weeks. At the end of the treatment, the patients undergo examination to determine the level of improvement.

### Example 3

Patients with ulcerative colitis are investigated. All patients are well-fed and well-hydrated at the time of the study. Diagnoses are conducted through endoscopic, radiologic, and histologic studies. Patients are characterized as having inactive, mild, moderately or severely active disease. Patients having inactive disease are defined as less than two bowel movements each day with no blood or mucus per rectum, and no visible inflammation as determined through sigmoidoscopy. Patients having mild disease are defined as less than four bowel movements per day accompanied by blood and mucus, and outer inflammation as shown by sigmoidoscopy. Patients having moderately or severely active disease are defined as greater than four bowel movements per day associated with cramping, but with evidence of fever or systemic signs of the disease found only in severe cases. Body mass index (BMI) was determined as weight (kg)/height (m)².

Tissue samples, which are freshly resected from the patients, are obtained.

The patients are divided into equal groups and are administered orally with about 50 mg, 100 mg or 200 mg per day of sulodexide such as VESSEL DUE F® for about 10 to 16 weeks. Alternatively, the patients are divided into equal groups and are administered parenterally with an injectable dextrose solution comprising 5 mg/day, 10 mg/day or 20 mg/day of sulodexide such as VESSEL DUE F® for two weeks. At the end of the treatment, the patients undergo examination to determine the level of improvement.

## Claims

1. A pharmaceutical composition comprising between 60% to 90% iduronylglycosaminoglycan sulfate or a pharmaceutically acceptable salt, solvate, hydrate or clathrate thereof, and between 10% to 40% dermatan sulfate or a pharmaceutically acceptable salt, solvate, hydrate or clathrate thereof, and an active ingredient selected from:
a) steroids, aminosalicylates, short-chain fatty acids, thioguanine derivatives, antibiotics, biological agents, antidepressants, pain relievers, and mixtures thereof, or
b) mesalamine, hydrocortisone, prednisolone, azathioprine, 6-MP, cyclosporine, methotrexate, metronidazole, ciprofloxacin, nicotine, penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin, imipramine, amitriptyline, desipramine, nortriptyline, doxepin, protriptyline, trimipramine, maprotiline, amoxapine, trazodone, bupropion, chlomipramine, fluoxetine, sertraline, paroxetine, fluvoxamine, nefazadone, venlafaxine, mirtazapine, monoamine oxidase inhibitors, phenelzine, tranylcypromine, acetaminophen, ibuprofen, and acetylsalicylic acid, naproxen, fenoprofen, indomethacin, ketorolac, thalidomide, infliximab, CDP571, IL-10, IL-11, ISIS-2302, anti-α4-antibody, Nissle 1917 and mixtures thereof,
for use in a method of preventing, reducing or eliminating one or more symptoms of inflammatory bowel disease in a patient wherein said pharmaceutical composition is to be administered to said patient in an amount sufficient to inhibit, reduce or eliminate one or more symptoms of inflammatory bowel disease.

2. A pharmaceutical composition as claimed in claim 1, wherein said composition comprises sulodexide or a pharmaceutically acceptable salt, solvate, hydrate or clathrate thereof.

3. A pharmaceutical composition as claimed in claim 1 or 2, wherein said composition is for oral, parenteral, transdermal or rectal administration.

4. A pharmaceutical composition as claimed in any one of claims 1 to 3, wherein said inflammatory bowel disease is Crohn's disease and/or ulcerative colitis.

5. A pharmaceutical composition as claimed in claim 1 or 2, wherein said composition is in the form of a tablet, capsule, suppository, solution, emulsion, suspension, aerosol spray, cachet, powder, transdermal patch, topical cream, lotion, ointment or gel.

6. A pharmaceutical composition as claimed in any one of claims 1 to 4, wherein said composition further comprises one or more excipients.

7. A pharmaceutical composition as claimed in any one of claims 1 to 5, wherein said composition further comprises one or more additional active ingredients and all active ingredients of the composition are to be administered in an amount sufficient to inhibit, reduce or eliminate one or more symptoms of inflammatory bowel disease.

8. A kit comprising:
a first pharmaceutical composition which comprises from 60% to 90% iduronylglycosaminoglycan sulfate or a pharmaceutically acceptable salt, solvate, hydrate, or clathrate thereof, and between 10% to 40% dermatan sulfate, or a pharmaceutically acceptable salt, solvate, hydrate, or clathrate thereof, and
a second pharmaceutical composition which comprises one or more additional active ingredients,
for use in a method of preventing, reducing or eliminating one or more symptoms of inflammatory bowel disease in a patient, wherein said first and second pharmaceutical compositions are to be administered to said patient in an amount sufficient to inhibit, reduce or eliminate one or more symptoms of inflammatory bowel disease.

9. A kit as claimed in claim 8, wherein said first and second pharmaceutical compositions are for separate administration and said administrations are temporally spaced apart by at least about two minutes.

10. Use of a composition comprising from 60% to 90% iduronylglycosaminoglycan sulfate or a pharmaceutically acceptable salt, solvate, hydrate or clathrate thereof, and between 10% to 40% dermatan sulfate or a pharmaceutically acceptable salt, solvate, hydrate or clathrate thereof, for the preparation of a medicament for inhibiting, reducing or eliminating one or more symptoms of inflammatory bowel disease.

11. A use as claimed in claim 10, wherein said composition comprises sulodexide or a pharmaceutically acceptable salt, solvate, hydrate or clathrate thereof.

12. A use as claimed in claim 10 or 11, wherein said medicament is adapted for oral, parenteral, transdermal or rectal administration.

13. A use as claimed in any one of claims 10 to 13, wherein said inflammatory bowel disease is Crohn's disease and/or ulcerative colitis.

14. A use as claimed in any one of claims 10 to 14, wherein said medicament further comprises an active ingredient selected from:
a) steroids, aminosalicylates, short-chain fatty acids, thioguanine, derivatives, antibiotics, biological agents, antidepressants and pain relievers, and mixtures thereof, or
b) mesalamine, hydrocortisone, prednisolone, azathioprine, 6-MP, cyclosporine, methotrexate, metronidazole, ciprofloxacin, nicotine, penicillins, cephalosporins, vancomycin, bacitracin, macrolides, lincosamides, chloramphenicol, tetracyclines, aminoglycosides, mupirocin, sulfonamides, trimethoprim, rifampin, metronidazole, quinolones, novobiocin, polymyxins, gramicidin, imipramine, amitriptyline, desipramine, nortriptyline, doxepin, protriptyline, trimipramine, maprotiline, amoxapine, trazodone, bupropion, chlomipramine, fluoxetine, sertraline, paroxetine, fluvoxamine, nefazadone, venlafaxine, mirtazapine, monoamine oxidase inhibitors, phenelzine, tranylcypromine, acetaminophen, ibuprofen, and acetylsalicylic acid, naproxen, fenoprofen, indomethacin, ketorolac, thalidomide, infliximab, CDP571, IL-10, IL-11, ISIS-2302, anti-α4-antibody, Nissle 1917 and mixtures thereof.

15. A use as claimed in any one of claims 10 to 14, wherein said medicament is to be administered in order to provide 20 lipoprotein lipase reducing agent units (L.R.U.) to 100,000 L.R.U. of said composition, per day.

16. A use as claimed in claim 11, wherein said medicament is to be administered in order to provide 20 L.R.U. to 100,000 L.R.U. of sulodexide, or a pharmaceutically acceptable salt, solvate, hydrate or clathrate thereof, per day.

17. A use as claimed in claim 10 or 11, wherein said medicament is to be administered:
a) parenterally, in order to provide 20 L.R.U. to 1,000 L.R.U., of said composition, per day;
b) orally, in order to provide 200 L.RU. to 10,000 L.R.U., of said composition, per day;
c) rectally, in order to provide 2000 L.R.U. to 100,000 L.R.U., of said composition, per day;
d) parenterally, in order to provide 2 mg to 100 mg of said composition, per day, or
e) orally, in order to provide 20 mg to 1000 mg of said composition, per day, or
f) rectally, in order to provide 200 mg to 10,000 mg of said composition, per day.

18. A use as claimed in claim 10 or 11, wherein said medicament is in the form of a tablet, capsule, suppository, solution, emulsion, suspension, aerosol spray, cachet, powder, transdermal patch, topical cream, lotion, ointment or gel.

19. A use as claimed in any one of claims 10 to 18, wherein said medicament further comprises one or more excipients.

## Patentansprüche

1. Pharmazeutische Zusammensetzung umfassend 60 % bis 90 % Iduronylglykosaminglykansulfat oder ein pharmazeutisch akzeptables Salz, Solvat, Hydrat oder Clathrat desselben und 10 % bis 40 % Dermatansulfat oder ein pharmazeutisch akzeptables Salz, Solvat, Hydrat oder Clathrat desselben und einen aktiven Bestandteil ausgewählt unter:
a) Steroiden, Aminosalicylaten, kurzkettigen Fettsäuren, Thioguaninderivaten, Antibiotika, biologischen Mitteln, Antidepressiva, schmerzlindernden Mitteln und Mischungen derselben oder
b) Mesalamin, Hydrocortison, Prednisolon, Azathioprin, 6-MP, Cyclosporin, Methotrexat, Metronidazol, Ciprofloxacin, Nicotin, Penicillinen, Cephalosporinen, Vancomycin, Bacitracin, Macroliden, Lincosamiden, Chloramphenicol, Tetracyclinen, Aminoglycosiden, Mupirocin, Sulfonamiden, Trimethoprim, Rifampin, Metronidazol, Chinolonen, Novobiocin, Polymyxinen, Gramicidin, Imipramin, Amitriptylin, Desipramin, Nortriptylin, Doxepin, Protriptylin, Trimipramin, Maprotilin, Amoxapin, Trazodon, Bupropion, Chlomipramin, Fluoxetin, Sertralin, Paroxetin, Fluvoxamin, Nefazodon, Venlafaxin, Mirtazapin, Monoaminoxidaseinhibitoren, Phenelzin, Tranylcypromin, Acetaminophen, Iboprofen und Acetylsalicylsäure, Naproxen, Fenoprofen, Indomethacin, Ketorolac, Thalidomid, Infliximab, CDP571, IL-10, IL-11, ISIS-2302, Anti-a4-Antikörper, Nissle 1917 und Mischungen derselben zur Verwendung bei einer Methode zum Verhindern, Reduzieren oder Eliminieren eines oder mehrerer Symptome der entzündlichen Darmerkrankung bei einem Patienten, wobei die pharmazeutisch Zusammensetzung dem Patienten in einer Menge verabreicht werden soll, die ausreicht, um ein oder mehrere Symptome der entzündlichen Darmerkrankung zu hemmen, reduzieren oder eliminieren.

2. Pharmazeutische Zusammensetzung nach Anspruch 1, wobei die Zusammensetzung Sulodexid oder ein pharmazeutisch akzeptables Salz, Solvat, Hydrat oder Clathrat desselben umfasst.

3. Pharmazeutischee Zusammensetzung nach Anspruch 1 oder 2, wobei die Zusammensetzung für die orale, parenterale, transdermale oder rektale Verabreichung bestimmt ist.

4. Pharmazeutische Zusammensetzung nach einem der Ansprüche 1 bis 3, wobei die entzündliche Darmerkrankung die Crohnsche Krankheit und/oder Colitis ulcerosa ist.

5. Pharmazeutische Zusammensetzung nach Anspruch 1 oder 2, wobei die Zusammensetzung in Form einer Tablette, Kapsel, eines Zäpfchens, einer Lösung, Emulsion, Suspension, eines Aerosolsprays, einer Oblatenkapsel, eines Pulvers, transdermalen Pflasters, einer topischen Creme, Lotion, Salbe oder eines Gels vorliegt.

6. Pharmazeutische Zusammensetzung nach einem der Ansprüche 1 bis 4, wobei die Zusammensetzung des Weiteren ein oder mehrere Vehikel umfasst.

7. Pharmazeutisch Zusammensetzung nach einem der Ansprüche 1 bis 5, wobei die Zusammensetzung des Weiteren einen oder mehrere zusätzliche aktive Bestandteile umfasst und alle aktiven Bestandteile der Zusammensetzung in einer Menge verabreicht werden sollen, die ausreicht, um ein oder mehrere Symptome der entzündlichen Darmerkrankung zu hemmen, reduzieren oder eliminieren.

8. Kit umfassend:
eine erste pharmazeutische Zusammensetzung, die 60 % bis 90 % Iduronylglykosaminglykansulfat oder ein pharmazeutisch akzeptables Salz, Solvat, Hydrat oder Clathrat desselben und 10 % bis 40 % Dermatansulfat oder ein pharmazeutisch akzeptables Salz, Solvat, Hydrat oder Clathrat desselben umfasst und
eine zweite pharmazeutische Zusammensetzung, die einen oder mehrere zusätzliche aktive Bestandteile umfasst,
zur Verwendung bei einer Methode zum Verhindern, Reduzieren oder Eliminieren eines oder mehrerer Symptome der entzündlichen Darmerkrankung bei einem Patienten, wobei die erste und zweite pharmazeutische Zusammensetzung dem Patienten in einer Menge verabreicht werden sollen, die ausreicht, um ein oder mehrere Symptome der entzündlichen Darmerkrankung zu hemmen, reduzieren oder eliminieren.

9. Kit nach Anspruch 8, wobei die erste und zweite pharmazeutische Zusammensetzung für die getrennte Verabreichung bestimmt sind und die Verabreichungen mindestens ca. zwei Minuten zeitlich getrennt sind.

10. Verwendung einer Zusammensetzung umfassend 60 % bis 90 % Iduronylglykosaminglykansulfat oder ein pharmazeutisch akzeptables Salz, Solvat, Hydrat oder Clathrat desselben und 10 % bis 40 % Dermatansulfat oder ein pharmazeutisch akzeptables Salz, Solvat, Hydrat oder Clathrat desselben für die Zubereitung eines Medikaments zum Verhindern, Reduzieren oder Eliminieren eines oder mehrerer Symptome der entzündlichen Darmerkrankung.

11. Verwendung nach Anspruch 10, wobei die Zusammensetzung Sulodexid oder ein pharmazeutisch akzeptables Salz, Solvat, Hydrat oder Clathrat desselben umfasst.

12. Verwendung nach Anspruch 10 oder 11, wobei das Medikament für die orale, parenterale, transdermale oder rektale Verabreichung geeignet ist.

13. Verwendung nach einem der Ansprüche 10 bis 13, wobei die entzündliche Darmerkrankung die Crohnsche Krankheit und/oder Colitis ulcerosa ist.

14. Verwendung nach einem der Ansprüche 10 bis 14, wobei das Medikament des Weiteren einen aktiven Bestandteil umfasst ausgewählt unter:
a) Steroiden, Aminosalicylaten, kurzkettigen Fettsäuren, Thioguaninderivaten, Antibiotika, biologischen Mitteln, Antidepressiva, schmerzlindernden Mitteln und Mischungen derselben oder
b) Mesalamin, Hydrocortison, Prednisolon, Azathioprin, 6-MP, Cyclosporin, Methotrexat, Metronidazol, Ciprofloxacin, Nicotin, Penicillinen, Cephalosporinen, Vancomycin, Bacitracin, Macroliden, Lincosamiden, Chloramphenicol, Tetracyclinen, Aminoglycosiden, Mupirocin, Sulfonamiden, Trimethoprim, Rifampin, Metronidazol, Chinolonen, Novobiocin, Polymyxinen, Gramicidin, Imipramin, Amitriptylin, Desipramin, Nortriptylin, Doxepin, Protriptylin, Trimipramin, Maprotilin, Amoxapin, Trazodon, Bupropion, Chlomipramin, Fluoxetin, Sertralin, Paroxetin, Fluvoxamin, Nefazadon, Venlafaxin, Mirtazapin, Monoaminoxidaseinhibitoren, Phenelzin, Tranylcypromin, Acetaminophen, Iboprofen und Acetylsalicylsäure, Naproxen, Fenoprofen, Indomethacin, Ketorolac, Thalidomid, Infliximab, CDP571, IL-10, IL-11, ISIS-2302, Anti-α4-Antikörper, Nissle 1917 und Mischungen derselben.

15. Verwendung nach einem der Ansprüche 10 bis 14, wobei das Medikament verabreicht werden soll, um 20 Lipoproteinlipase-Reduziermitteleinheiten (LRE) bis 100.000 LRE der Zusammensetzung pro Tag zu liefern.

16. Verwendung nach Anspruch 11, wobei das Medikament verabreicht werden soll, um 20 LRE bis 100.000 LRE Sulodexid oder pharmazeutisch akzeptables Salz, Solvat, Hydrat oder Clathrat desselben pro Tag zu liefern.

17. Verwendung nach Anspruch 10 oder 11, wobei das Medikament wie folgt verabreicht werden soll:
a) parenteral, um 20 LRE bis 1.000 LRE der Zusammensetzung pro Tag zu liefern;
b) oral, um 200 LRE bis 10.000 LRE der Zusammensetzung pro Tag zu liefern;
c) rektal, um 2000 LRE bis 100.000 LRE der Zusammensetzung pro Tag zu liefern;
d) parenteral, um 2 mg bis 100 mg der Zusammensetzung pro Tag zu liefern;
e) oral, um 20 mg bis 1000 mg der Zusammensetzung pro Tag zu liefern;
f) rektal, um 200 mg bis 10.000 mg der Zusammensetzung pro Tag zu liefern.

18. Verwendung nach Anspruch 10 oder 11, wobei das Medikament in Form einer Tablette, Kapsel, eines Zäpfchens, einer Lösung, Emulsion, Suspension, eines Aerosolsprays, einer Oblatenkapsel, eines Pulvers, transdermalen Pflasters, einer topischen Creme, Lotion, Salbe oder eines Gels vorliegt.

19. Verwendung nach einem der Ansprüche 10 bis 18, wobei das Medikament des Weiteren ein oder mehrere Vehikel umfasst.

## Revendications

1. Composition pharmaceutique qui comprend de 60 % à 90 % de sulfate d'iduronyl-glycosaminoglycane ou d'un sel, solvate, hydrate ou clathrate pharmaceutiquement acceptable de celui-ci, de 10 % à 40 % de sulfate de dermatane ou d'un sel, solvate, hydrate ou clathrate pharmaceutiquement acceptable de celui-ci et un principe actif sélectionné parmi les suivants :
a) stéroïdes, aminosalicylates, acides gras à chaîne courte, dérivés de la thioguanine, antibiotiques, agents biologiques, antidépresseurs, agents soulageant la douleur et mélanges de ces composés, ou
b) mésalamine, hydrocortisone, prednisolone, azathioprine, 6-MP, cyclosporine, méthotrexate, métronidazole, ciprofloxacine, nicotine, pénicillines, céphalosporines, vancomycine, bacitracine, macrolides, lincosamides, chloramphénicol, tétracyclines, aminoglycosides, mupirocine, sulfonamides, triméthoprime, rifampine, métronidazole, quinolones, novobiocine, polymyxines, gramicidine, imipramine, amitriptyline, désipramine, nortriptyline, doxépine, protriptyline, trimipramine, maprotiline, amoxapine, trazodone, bupropion, clomipramine, fluoxétine, sertraline, paroxétine, fluvoxamine, néfazadone, venlafaxine, mirtazapine, inhibiteurs de la monoamine-oxydase, phénelzine, tranylcypromine, acétaminophène, ibuprofène, acide acétylsalicylique, naproxène, fénoprofène, indométhacine, kétorolac, thalidomide, infliximab, CDP571, IL-10, IL-11, ISIS-2302, anticorps anti-α4, Nissle 1917 et des mélanges de ces composés,
ladite composition pharmaceutique étant destinée à être utilisée dans une méthode visant à prévenir, réduire ou éliminer un ou plusieurs des symptômes d'une affection intestinale inflammatoire chez un patient et devant être administrée audit patient en une quantité suffisante pour inhiber, réduire ou éliminer un ou plusieurs symptômes d'une affection intestinale inflammatoire.

2. Composition pharmaceutique selon la revendication 1, qui comprend du sulodexide ou un sel, solvate, hydrate ou clathrate pharmaceutiquement acceptable de ce composé.

3. Composition pharmaceutique selon la revendication 1 ou 2, qui se prête à une administration par voie orale, parentérale, transdermique ou rectale.

4. Composition pharmaceutique selon l'une quelconque des revendications 1 à 3, où ladite affection intestinale inflammatoire est la maladie de Crohn et/ou la rectocolite hémorragique.

5. Composition pharmaceutique selon la revendication 1 ou 2, qui se présente sous la forme d'un comprimé, d'une gélule, d'un suppositoire, d'une solution, d'une émulsion, d'une suspension, d'une bouffée d'aérosol, d'un cachet, d'une poudre, d'un timbre transdermique, d'une crème à usage local, d'une lotion, d'une pommade ou d'un gel.

6. Composition pharmaceutique selon l'une quelconque des revendications 1 à 4, qui comprend également un ou plusieurs excipients.

7. Composition pharmaceutique selon l'une quelconque des revendications 1 à 5 qui comprend également un ou plusieurs principes actifs additionnels, tous les principes actifs de la composition étant destinés à être administrés en une quantité suffisante pour inhiber, réduire ou éliminer un ou plusieurs symptômes d'une affection intestinale inflammatoire.

8. Nécessaire qui comporte :
une première composition pharmaceutique qui comprend de 60 % à 90 % de sulfate d'iduronyl-glycosaminoglycane ou d'un sel, solvate, hydrate ou clathrate pharmaceutiquement acceptable de celui-ci et de 10 % à 40 % de sulfate de dermatane ou d'un sel, solvate, hydrate ou clathrate pharmaceutiquement acceptable de celui-ci, et
une seconde composition pharmaceutique qui comprend un ou plusieurs principes actifs additionnels,
destinées à être utilisées dans une méthode visant à prévenir, réduire ou éliminer un ou plusieurs des symptômes d'une affection intestinale inflammatoire chez un patient, lesdites première et seconde compositions pharmaceutiques étant administrées audit patient en des quantités suffisantes pour inhiber, réduire ou éliminer un ou plusieurs symptômes d'une affection intestinale inflammatoire.

9. Nécessaire selon la revendication 8, où les dites première et seconde compositions pharmaceutiques sont destinées à être administrées séparément de façon à ce que l'intervalle ménagé entre lesdites administrations soit de deux minutes au moins.

10. Utilisation d'une composition qui comprend de 60 % à 90 % de sulfate d'iduronyl-glycosaminoglycane ou d'un sel, solvate, hydrate ou clathrate pharmaceutiquement acceptable de celui-ci et de 10 % à 40 % de sulfate de dermatane ou d'un sel, solvate, hydrate ou clathrate pharmaceutiquement acceptable de celui-ci dans la préparation d'un médicament conçu pour inhiber, réduire ou éliminer un ou plusieurs symptômes d'une affection intestinale inflammatoire.

11. Utilisation selon la revendication 10, où ladite composition comprend du sulodexide ou un sel, solvate, hydrate ou clathrate pharmaceutiquement acceptable de ce composé.

12. Utilisation selon la revendication 10 ou 11, où ledit médicament se prête à une administration par voie orale, parentérale, transdermique ou rectale.

13. Utilisation selon l'une quelconque des revendications 10 à 13, où ladite affection intestinale inflammatoire est la maladie de Crohn et/ou la rectocolite hémorragique.

14. Utilisation selon l'une quelconque des revendications 10 à 14, où ledit médicament comprend également des principes actifs sélectionnés parmi les suivants :
a) stéroïdes, aminosalicylates, acides gras à chaîne courte, dérivés de la thioguanine, antibiotiques, agents biologiques, antidépresseurs, agents soulageant la douleur et mélanges de ces composés ou
b) mésalamine, hydrocortisone, prednisolone, azathioprine, 6-MP, cyclosporine, méthotrexate, métronidazole, ciprofloxacine, nicotine, pénicillines, céphalosporines, vancomycine, bacitracine, macrolides, lincosamides, chloramphénicol, tétracyclines, aminoglycosides, mupirocine, sulfonamides, triméthoprime, rifampine, métronidazole, quinolones, novobiocine, polymyxines, gramicidine, imipramine, amitriptyline, désipramine, nortriptyline, doxépine, protriptyline, trimipramine, maprotiline, amoxapine, trazodone, bupropion, clomipramine, fluoxétine, sertraline, paroxétine, fluvoxamine, néfazadone, venlafaxine, mirtazapine, inhibiteurs de la monoamine-oxydase, phénelzine, tranylcypromine, acétaminophène, ibuprofène, acide acétylsalicylique, naproxène, fénoprofène, indométhacine, kétorolac, thalidomide, infliximab, CDP571, IL-10, IL-11, ISIS-2302, anticorps anti-α4, Nissle 1917 et des mélanges de ces composés.

15. Utilisation selon l'une quelconque des revendications 10 à 14, où ledit médicament doit être administré de façon à fournir de 20 L.R.U (unités d'un agent réduisant l'activité lipoprotéine-lipase) à 100 000 L.R.U. de ladite composition par jour.

16. Utilisation selon la revendication 11, où ledit médicament doit être administré de façon à fournir de 20 L.R.U. à 100 000 L.R.U. par jour de sulodexide ou d'un sel, solvate, hydrate ou clathrate pharmaceutiquement acceptable de ce composé.

17. Utilisation selon la revendication 10 ou 11, où ledit médicament doit être administré :
a) par voie parentérale, de façon à fournir de 20 L.R.U. à 1 000 L.R.U. de ladite composition par jour ;
b) par voie orale, de façon à fournir de 200 L.R.U. à 10 000 L.R.U. de ladite composition par jour ;
a) par voie rectale, de façon à fournir de 2 000 L.R.U. à 100 000 L.R.U. de ladite composition par jour ;
d) par voie parentérale, de façon à fournir de 2 mg à 100 mg de ladite composition par jour, ou
e) par voie orale, de façon à fournir de 20 mg à 1 000 mg de ladite composition par jour, ou
a) par voie rectale, de façon à fournir de 200 mg à 10 000 mg de ladite composition par jour.

18. Utilisation selon la revendication 10 ou 11, où ledit médicament se présente sous la forme d'un comprimé, d'une gélule, d'un suppositoire, d'une solution, d'une émulsion, d'une suspension, d'une bouffée d'aérosol, d'un cachet, d'une poudre, d'un timbre transdermique, d'une crème à usage local, d'une lotion, d'une pommade ou d'un gel.

19. Utilisation selon l'une quelconque des revendications 10 à 18, où ledit médicament comprend également un ou plusieurs excipients.
